(19) Europäisches Patentamt
European Patent Office
Office européen des brevets

(11) Numéro de publication: **0 167 419**
**A1**

(12) # DEMANDE DE BREVET EUROPEEN

(21) Numéro de dépôt: 85400866.1

(22) Date de dépôt: 03.05.85

(51) Int. Cl.⁴: **A 01 N 43/36**

(30) Priorité: 04.05.84 FR 8406980

(43) Date de publication de la demande: 08.01.86 Bulletin 86/2

(84) Etats contractants désignés: BE CH DE FR GB IT LI NL

(71) Demandeur: Etablissement Public dit: CENTRE NATIONAL DE LA RECHERCHE SCIENTIFIQUE (CNRS)
15, Quai Anatole France
F-75007 Paris(FR)

(72) Inventeur: Clement, Jean-Luc
15, rue de Monttessuy
F-75007 Paris(FR)

(72) Inventeur: Lemaire, Michèle
48, avenue Victor Hugo
92140 Clamart(FR)

(72) Inventeur: Lange, Catherine
48, rue de Dunkerque
F-75009 Paris(FR)

(72) Inventeur: Lhommet, Gérard
1, rue de Sévigné
F-94500 Champigny S/Marne(FR)

(72) Inventeur: Celerier, Jean-Pierre
80, boulevard de Reuilly
F-75012 Paris(FR)

(72) Inventeur: Basselier, Jean-Jacques
6, boulevard Guynemer
F-91170 Viry Chatillon(FR)

(72) Inventeur: Cassier, Pierre
22, rue de Balzac
F-92600 Asnieres(FR)

(74) Mandataire: Nony, Michel
Cabinet Nony 29, rue Cambacérès
F-75008 Paris(FR)

(54) **Compositions insecticides et/ou acaricides, notamment termicides, à base de dérivés 2,5-disubtitués de la pyrrolidine et/ou de la pryrroline-1.**

(57) Composition insecticide et/ou acaricide et procédé de destruction des insectes notamment des termites.

La composition contient dans un véhicule approprié, en tant que substance active, au moins un composé correspondant à la formule générale suivante :

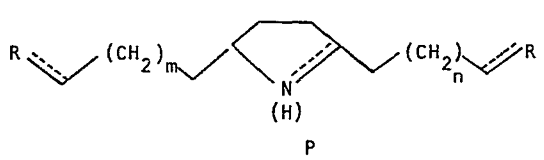

(I)

dans laquelle,

R et R', identiques ou différents, représentent un radical méthyle ou méthylène,

m et n, identiques ou différents représentent 0 ou un nombre entier de 1 à 12,

et p est égal à 0 lorsque l'hétérocycle est la pyrroline-1 ou p est égal à 1 lorsque l'hétérocycle est la pyrrolidine,

et, les isomères et sels desdits composés

EP 0 167 419 A1

Compositions insecticides et/ou acaricides, notamment termicides, à base de dérivés 2,5-disubstitués de la pyrrolidine et/ou de la pyrroline-1.

La présente invention a pour objet de nouvelles compositions insecticides et/ou acaricides notamment termicides contenant, en tant que substance active, des dérivés 2,5-disubstitués de la pyrrolidine et/ou de la pyrroline-1.

Les termites du genre Reticulitermes ainsi que d'autres insectes tels que les punaises (Piesma quadrate ou Cimex lectularium) les criquets (Locusta migratoria, Gryllus domesticus) et les diptères ainsi que les acariens sont particulièrement nuisibles.

En vue de lutter contre ces insectes, différentes substances insecticides ont été jusqu'ici proposées, il s'agit notamment du DTT et de produits apparentés, de composés organochlorés ou phosphorés, de méthyl-carbamates et de pyréthrinoïdes comme la deltaméthrine, cette dernière substance s'étant avérée être un puissant insecticide à très faible dose.

Jusqu'à ce jour, il n'a pas été possible de lutter de façon efficace et sélective à l'encontre des termites qui provoquent, comme ceci est bien connu, de nombreux dégats dans les structures et objets en bois, notamment dans certaines régions où ces insectes prolifèrent.

On a maintenant constaté qu'il était possible de lutter avec une très bonne efficacité non seulement à l'égard des isoptères (termites) mais également à l'égard d'autres insectes appartenant aux groupes des orthoptères (criquets), hétéroptères (punaises) et diptères ainsi qu'à l'égard des acariens en utilisant en tant que substance active des dérivés 2,5-disubstitués de la pyrrolidine et/ou de la pyrroline-1, notamment des dérivés 2,5-dialkyles et 2,5-dialkényles.

Certains de ces dérivés de la pyrrolidine et de la pyrroline-1 ont déjà été décrits dans la littérature comme constituants de glandes à poison de certaines espèces de fourmis du genre Monomorium, ces dérivés ayant jusqu'à ce jour été uniquement considérés comme répulsifs à l'égard des espèces compétitrices ou comme des constituants de la phéromone de piste.

On peut à cet égard faire référence aux publications suivantes:

1° - T.H.JOHNES et al, Journ. of chemical Ecology, 8, 1, 1982 p. 285,

2° - C.B.URBANI et al. Envir. Ent. 3, 1974 p. 755,

3° - F.W. HOWARD et al. J. Georgia. Ent. Soc. 14, 1979, p.259,

4° - F.J. RITER et al. Neth. J. Zool. 25, 1975, p.261, et

5° - T.H. JOHNES et al. Tétrahedron. Lett. 21, 1980, p.789.

La présente invention repose sur la découverte surprenante que ces dérivés 2,5-disubstitués de la pyrrolidine et de la pyrroline-1 ainsi que d'autres dérivés apparentés possèdent un excellent pouvoir insecticide à

l'égard d'une grande variété d'insectes notamment à l'égard des termites du genre Reticulitermes ainsi qu'une action acaricide puissante.

La présente invention a donc pour objet, à titre de produit industriel nouveau, une composition insecticide et/ou acaricide notamment termicide, contenant, dans un véhicule approprié, en tant que substance active, au moins un composé correspondant à la formule générale suivante :

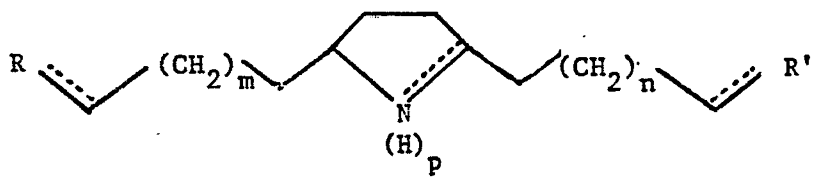 (I)

dans laquelle,

R et R', identiques ou différents, représentent un radical méthyle ou méthylène,

m et n, identiques ou différents, représentent 0 ou un nombre entier de 1 à 12, m + n étant de préférence $\geqslant 4$,

et p est égal à 0 lorsque l'hétérocycle est la pyrroline-1 ou p est égal à 1 lorsque l'hétérocycle est la pyrrolidine,

et, les isomères et sels desdits composés de formule (I).

Selon une première forme de réalisation, les composés actifs de la composition sont des dérivés 2,5-disubstitués de la pyrrolidine et correspondent à la formule suivante :

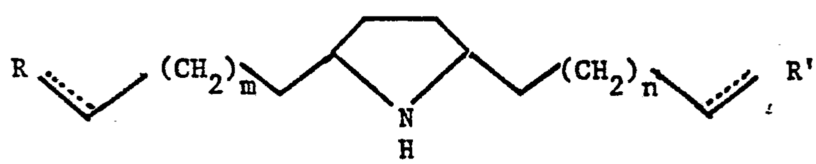 (II)

dans laquelle :

R, R', m et n ont les mêmes significations que ci-dessus.

Parmi les composés correspondant à la formule (II), on peut citer les suivants :

(Hexène-5 yl)-2 (nonène-8 yl)-5 pyrrolidine,
Di(nonène-8 yl)-2,5 pyrrolidine,
Di(decène-9 yl)-2,5 pyrrolidine,
(Hexène-5 yl)-2 nonyl-5 pyrrolidine,
Hexyl-2-nonyl-5 pyrrolidine,
Hexyl-2-pentyl-5 pyrrolidine,
Di hexyl-2,5 pyrrolidine, et
(Hexène-5 yl)-2 pentyl-5 pyrrolidine.

Selon une deuxième forme de réalisation, les composés actifs de la composition sont des dérivés 2,5 disubstitués de la pyrroline-1 et correspondent à la formule suivante :

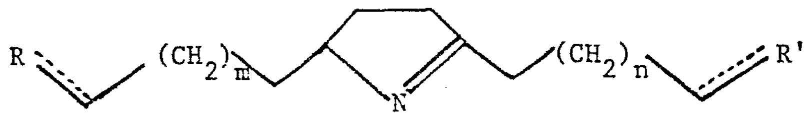 (III)

dans laquelle :

R, R', m et n ont les mêmes significations que ci-dessus.

Parmi les composés correspondant à la formule (III), on peut citer les suivants :

Dihydro-3,4 (hexène-5 yl)-2 (nonène-8 yl)-5, 2 H pyrrole, ou
(Hexène-5 yl)-2 (nonène-8 yl)-5 pyrroline-1 et
Dihydro-3,4 (hexène-5 yl)-2 nonyl-5, 2 H pyrrole ou
(Hexène-5 yl)-2 nonyl-5 pyrroline-1

Les essais effectués ont permis de montrer que la structure stéréochimique des composés actifs n'influait pas de façon sensible sur l'activité insecticide et/ou acaricide.

Les raisons de cette activité particulièrement puissante n'ont pu encore être mises en évidence de façon certaine mais on suppose que les substances agissent sur le système nerveux, l'action étant instantanée et brutale (Knock Down).

Après pulvérisation, les insectes restent immobilisés et meurent dans les douze heures suivantes. On suppose néanmoins que ces substances actives, du fait de leur structure, possèdent une bonne liposolubilité leur permettant de traverser facilement la cuticule des insectes.

Les essais de toxicité sur les vertébrés ont par ailleurs permis de montrer que ces substances n'étaient pas toxiques, les doses létales chez la souris par injection sous-cutanée étant supérieures à 100 mg/kg.

Parmi les substances particulièrement actives de formule (I) ci-dessus, on doit tout particulièrement mentionner les suivantes :

(Hexène-5 yl)-2 (nonène-8 yl)-5 pyrrolidine (composé A)
(Hexène-5 yl)-2 nonyl-5 pyrrolidine (composé B).

Les essais effectués sur différents types de termites européens du genre Reticulitermes à l'aide de ces deux composés ont donné les résultats suivants exprimés en µg de substance active par mg de termites.

| Dose létale 50 | Reticulitermes santonensis | Reticulitermes grassei | Reticulitermes banyulensis |
|---|---|---|---|
| Composé A | 0,14µg | 0,25µg | 0,18µg |
| Composé B | 1,30µg | 2,54µg | 1,99µg |

Comme ceci a été précisé ci-dessus, certains des composés de formule (I) ont déjà été décrits ainsi que leur méthode de synthèse.

Pour la synthèse des composés de formule (II) diverses méthodes peuvent être utilisées, notamment celles basées sur la réaction de HOFMAN-LOFFLER (D.J. PEDDER et al, Tetrahedron, 32, 2275 (1976), la réduction au borohydrure des pyrrolines correspondantes formées par le réarrangement de MUNDY (F.J. RITTER et al, Brevet US 4.075.320), l'alkylation directe de N-nitrosopyrrolidine (R.R. FRASER et al, Synthesis, 540, 1976), l'hydrogénation catalytique de pyrroles (T.H. JOHNES et al, Tetrahedron Letters 1031, 1979) et l'amination réductrice de dicétones-1,4.

Cette dernière méthode qui a été décrite par T.H. JOHNES et al, Tetrahedron Letters 789, (1980) est particulièrement avantageuse et a été utilisée pour la préparation des substances actives des compositions selon l'invention.

Les étapes de cette méthode peuvent être représentées à l'aide du schéma réactionnel suivant :

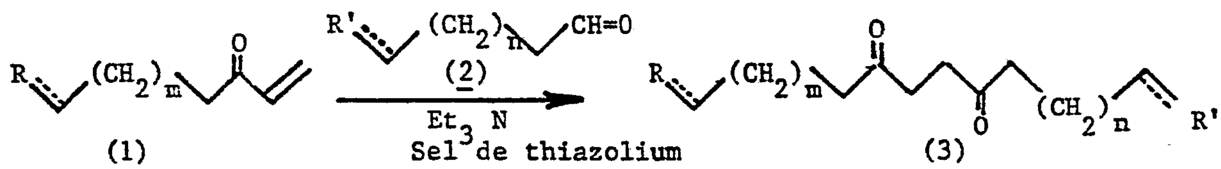


(1)                (3)

(3)

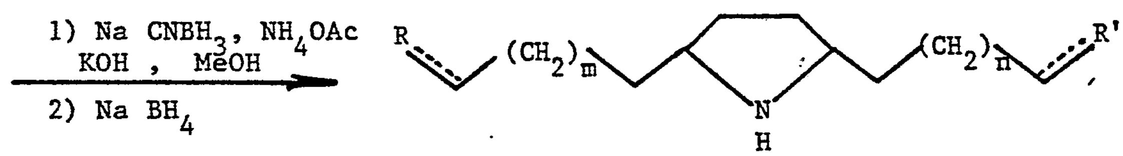


cis et/ou trans

(II)

Selon cette méthode, on fait réagir par condensation une cétone insaturée (1) avec un aldéhyde (2) en présence, comme catalyseur, d'un sel de thiazolium tel que par exemple le chlorure de 5-(2'-hydroxyethyl)-4-methyl -3-benzylthiazolium et en présence de triéthylamine. La réaction de condensation est effectuée à reflux sous azote et la dicétone obtenue (3) est isolée par distillation sous vide et/ou par recristallisation dans un solvant approprié.

Les 2,5-pyrrolidines de formule (II) sont obtenues par réaction d'amination réductrice des dicétones (3) en présence d'acétate d'ammonium, de potasse et de cyanoborohydrure de sodium dans le méthanol pendant environ 10 à 24 heures. Après la réaction, le mélange est agité pendant 2 à 3 heures avec

un léger excès de borohydrure de sodium et l'on isole selon les méthodes habituelles les 2,5-pyrrolidines de formule (II) avec un rendement compris entre 50 et 90 %.

Les dérivés de la pyrrolidine se présentent le plus souvent sous forme d'un liquide incolore contenant en mélange les isomères cis et trans.

Au cours de la réaction d'amination réductrice, il peut se former, au départ de certaines dicétones (3), les pyrroles correspondants mais en une quantité généralement inférieure à 15 %.

Les dérivés 2,5-disubstitués de la pyrroline-1 de formule (III) sont généralement obtenus à partir des pyrrolidines correspondantes par traitement à l'aide d'une solution méthanolique contenant un excès d'une solution à 5 % d'hypochlorite de sodium.

Après reflux pendant 3 à 5 heures avec un excès d'environ 10 fois la quantité requise d'hydroxyde de sodium, on obtient les pyrrolines de formule (III) sous forme d'un mélange d'isomères.

Bien qu'il ait été fait référence dans la formule (III) à un isomère particulier, il va de soi que lorsque les radicaux R et R' sont différents, les pyrrolines peuvent se présenter sous deux formes isomériques selon que la double liaison de l'hétérocycle est en position 2 ou 5.

Les cétones insaturées (1) sont généralement instables et sont obtenues en deux étapes selon le schéma réactionnel suivant :

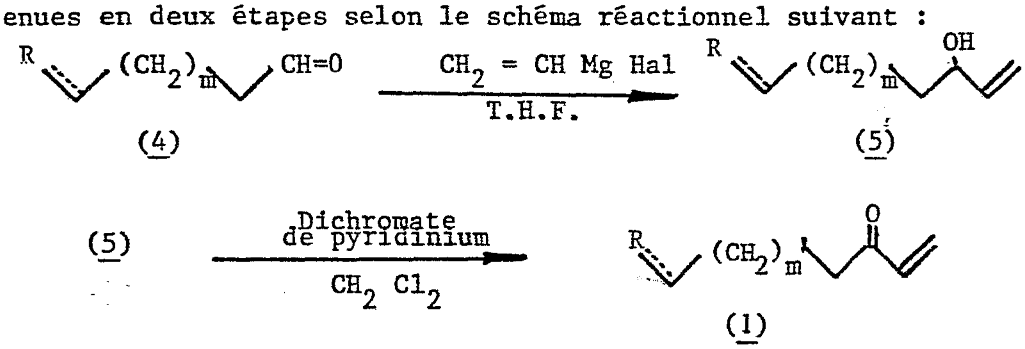


(4) (5)

(5) (1)

La première étape consiste à faire réagir le magnésien du chlorure ou bromure de vinyle dans le THF sur un aldéhyde saturé ou insaturé de formule (4) et à oxyder ensuite l'alcool secondaire allylique dans le chlorure de méthylène en présence de dichromate de pyridinium selon les méthodes décrites par F. BOHLMANN et al, Chem. Ber. 98 3010 (1965) et E.J. COREY et al, Tetrahedron, lett. 2647 (1975).

La plupart des cétones insaturées (1) sont instables et doivent être utilisées le plus rapidement possible après leur préparation.

METHODE GENERALE DE PREPARATION DES COMPOSES ACTIFS

A) Préparation des dicétones de formule (3)

0,1 Mole de cétone-éthylènique de formule (1), 0,1 Mole d'aldéhyde de formule (2) et 3,2 g de chlorure de 5-(2'-hydroxyéthyl)-4-méthyl-

3-benzylthiazolium sont chauffés au reflux et sous azote dans 18 ml de triéthylamine pendant 20 heures.

On refroidit puis ajoute 50 ml d'éther. On filtre le précipité, lave avec de petites portions d'éther. On réunit les phases éthérées, évapore le solvant puis distille sous vide la dicétone (3) obtenue.

Selon ce mode opératoire, on a obtenu les $\delta$ dicétones suivantes :

Nonadecadiène - 1,18 dione - 7,10

Rendement : 85 % F : 40°C (après distillation au KUGELROHR).

RMN à 250 $MH_z$. ($CDCl_3$).$\delta$ en ppm.

5,55 - 5,85 2 H ($CH_2$ = CH - ) ; 4,75 - 5,05.4 H ($\underline{CH_2}$ = CH - ) ; 2,60$\delta$ (4 H, CO-$\underline{CH_2}$-$\underline{CH_2}$-CO), 2,25 - 2,45 m 4H ($\underline{CH_2}$ CO), 1,90 - 2,05 m 4H ($\underline{CH_2}$ - CH = $CH_2$), 1,05 - 1,65 m 14 H ($\underline{CH_2}$ - alkyl).

Docosadiène - 1,21, dione - 10,13

Rendement : 50 % F ≅ 48°C (après distillation au KUGELROHR).

RMN à 250 $MH_z$ $\delta$ en ppm ($CDCl_3$)

5,70 - 5,90 m 2H ($CH_2$ = $\underline{CH}$ -) ; 4,90 - 5,10 m 4H($CH_2$ = CH) ;

2,58 s 4H (-CO$\underline{CH_2}$ $\underline{CH_2}$CO-)

2,45 4H t (CO $CH_2$ ...) ; 1,90 - 2,10 4H ($CH_2$ = CH - $\underline{CH_2}$-) ;

1,20-1,80m20H (-$CH_2$ - alkyl)

Tetracosadiène-1,23, Dione-11,14

Rendement : 55 % F = 72° C (Recristallisation dans le Méthanol)

IR($HCBr_3$)$\nu$ en $cm^{-1}$ : 3100, 3040, 1710, 1640, 1150

RMN à 60 MHz ($CDCl_3$)$\delta$ en ppm :

5,40 - 6,10 m, 2H ($CH_2$ = $\underline{CH}$-) ; 4,70 - 5,20 m, 4H ($\underline{CH_2}$ = CH-) ;

2,66 s, 4H (-CO-$\underline{CH_2}$-$\underline{CH_2}$-CO-) ; 2,25 - 2,60 m, 4H ($\underline{CH_2}$ CO) ;

1,85 - 2,20 m, 4H ($\underline{CH_2}$-CH = $CH_2$) ; 0,95 - 1,75 m, 24H ($CH_2$-alkyl).

Nonadécanedione-7,10

Rendement : 50 % $E_{0,01}$ 225° C (Après distillation au Kugelrohr)

RMN à 60 MHz ($CDCl_3$)$\delta$ en ppm :

2,65 s,4H (-CO-$\underline{CH_2}$-$\underline{CH_2}$-CO-) ; 2,30 - 2,60 m, 4H (-$\underline{CH_2}$-CO) ;

1,0 - 1,70 m, 22H ($CH_2$ alkyl) ; 0,70 - 0,95 m, 6H (-$CH_2$-$CH_2$-$\underline{CH_3}$).

Hexadecanedione-7,10

Rendement : 80 % $E_{0,01}$ ∿ 210° C (Après distillation au Kugelrohr)

IR (film)$\nu$ en $cm^{-1}$ : 3020, 1705, 1460, 1140

RMN à 60 MHz ($CDCl_3$) $\delta$ en ppm :

2,75 s, 4H (-CO-$\underline{CH_2}$ $CH_2$-CO-) ; 2,40 - 2,70 m, 4H (-$\underline{CH_2}$-CO) ;

1,10 - 1,80 m, 16H ($CH_2$ alkyl) ; 0,80 - 1,05 m, 6H (-$CH_2$-$CH_2$-$\underline{CH_3}$).

Les autres dicétones de formule (3) sont obtenues selon le même mode opératoire que celui décrit ci-dessus.

B) Préparation des 2,5-pyrrolidines de formule (II)

Une solution contenant 0,07 mole de dicétone de formule (3), 1 g de potasse pulvérisée, 5,5 g d'acétate d'ammonium et 5 g de cyanoborohydrure de sodium dans 150 ml de méthanol anhydre est agitée pendant 15 heures. On ajoute ensuite 0,1 Mole de borohydrure de sodium puis agite encore une heure pour terminer la réaction. On ajoute 100 ml d'eau, neutralise par une soluction d'HCl à 10 %, sature la solution par du carbonate de potassium puis extrait avec 4 fois 100 ml de $CH\ Cl_3$. On sèche les phases organiques réunies, évapore le solvant puis distille avec un Kugelrohr le liquide obtenu.

Selon ce mode opératoire, on a obtenu les composés actifs de formule (II) suivants :

<u>(Hexène-5 yl)-2 (nonène-8 yl)-5 pyrrolidine cis et trans.</u>

Rendement 90 % $E_{0,01}$ 190 - 210°C (Décomposition).

IR (film) en $cm^{-1}$ 3380 ; 3040 ; 1640, 1460, 980, 910.

RMN à 60 $MH_z$ ($CDCl_3$) $\delta$ en ppm.

5,30 - 6,0 m, 2H ($CH_2$ = <u>CH</u> - ) ; 4,70 - 5,10,m , 4H (<u>$CH_2$</u> = CH - ) ;
2,70 - 3,20,m 2H (-<u>CH</u> - NH - <u>CH</u> - ) ; 1,70, 2,40 m 8 H
(<u>$CH_2$</u> - CH = $CH_2$ et $CH_2$ du cycle),
1,9 - 2,1 m, 1H (N - H) 1,10 - 1,70, m, 18H (alkyl - $CH_2$ - ).

<u>Di-(nonène-8 yl)-2,5 pyrrolidine</u>

Rendement : 45%

RMN à 60 $MH_z$ $\delta$ en ppm ($CDCl_3$)

5,45 - 6,0 m 2H ($CH_2$ = <u>CH</u> - ) ; 4,75 - 5,20 m, 4H (<u>$CH_2$</u> = CH-) ;
2,75 - 3,20 m 2H (- CH - NH - CH -) ; 1,70 - 2,30 m 8H
($CH_2$ = CH - $CH_2$ - et $CH_2$ du cycle); 1,0 - 2,70 m 24 H (-$CH_2$- alkyl).

<u>Di (decène-9 yl)-2,5 pyrrolidine</u>

Rendement : 58 % F ≃ 74° C (Recristallisation dans l'Ether)

IR ($CHBr_3$)$\nu$ en $cm^{-1}$ : 3280, 3100, 3040, 1640, 1455, 1150

RMN à 60 MHz ($CDCl_3$) $\delta$ en ppm :

5,30 - 6,15 m, 2H ($CH_2$ = <u>CH</u>-) ; 4,75 - 5,25 m, 4H (<u>$CH_2$</u> = CH-) ;
3,20 - 3,80 m, 1H (NH) ; 2,80 - 3,30 m, 2H (-<u>CH</u>-NH-<u>CH</u>-) ;
1,75 - 2,30 m, 8H (<u>$CH_2$</u>-CH = $CH_2$ et $CH_2$ du cycle) ;
1,05 - 1,70 m, 24 H ($CH_2$ alkyl).

<u>Hexyl-2 nonyl-5 pyrrolidine</u>

Rendement : 50 % $E_{0,05}$ 200-205° C (Après distillation au Kugelrohr)

RMN à 60 MHz ($CDCl_3$) $\delta$ en ppm :

5,20 - 5,40 m, 1H (NH) ; 2,40 - 2,95 m, 2H (-<u>CH</u>-NH-<u>CH</u>-) ; 2,20 - 2,40 m, 4H ($CH_2$ du cycle) ; 1,0 - 1,80 m, 26H ($CH_2$ alkyl) ; 0,60 - 0,95 m, 6H ($-CH_2-CH_2-\underline{CH_3}$).

<u>Di hexyl-2,5 pyrrolidine</u>

Rendement : 60 % F 163-165° C (Recristallisation dans l'Ethanol)

IR ($HCBr_3$)$\nu$ en $cm^{-1}$ : 3380, 1580, 1460

RMN à 60 MHz ($CDCl_3$)$\delta$ en ppm :

3,20 - 3,60 m, 2H (-<u>CH</u>-NH-<u>CH</u>-) ; 1,90 - 2,0 m, 1H (NH) ; 1,75 - 2,30 m, 4H ($CH_2$ du cycle) ; 1,05 - 1,70 m, 20H ($CH_2$ alkyl) ; 0,80 - 1,10 m, 6H ($-CH_2-CH_2-\underline{CH_3}$).

Les autres composés actifs de formule (II) ont été obtenus selon le même mode opératoire que celui décrit ci-dessus.

Les compositions insecticides et/ou acaricides selon l'invention peuvent être mises en oeuvre sous une forme permettant une application commode. Par exemple, les substances actives peuvent être utilisées dans des compositions sous forme d'émulsions, de suspensions, de solutions, de poudres ou d'aérosols.

Pour la préparation des solutions pulvérisables, on peut utiliser par exemple des fractions d'huiles minérales distillant entre les hautes et moyennes températures, des huiles d'origine animale ou végétale, des hydrocarbures aliphatiques comme l'hexane ou le pentane, des hydrocarbures aromatiques comme les naphtalènes alkylés, le tétrahydronaphtalène, éventuellement avec emploi de mélange de xylènes, de cyclohexanols, de cétones, d'hydrocarbures halogénés comme le tri- et le tétrachloroéthane, le trichloroéthylène ou le tri- et le tétrachlorobenzène. On utilise de préférence des solvants organiques présentant un point d'ébullition supérieur à 100°C.

Les préparations peuvent également être aqueuses telles que des émulsions, pâtes ou poudres mouillables par addition d'eau.

Comme dispersants, on emploie des produits non-ioniques, par exemple des produits de condensation d'alcool aliphatique, d'amines ou d'acides carboxyliques, ayant un radical hydrocarboné à longue chaîne contenant entre 10 et 20 atomes de carbone avec de l'oxyde d'éthylène, comme le produit de condensation de l'alcool octadécylique avec 25 à 30 molécules d'oxyde d'éthylène, ou celui de l'acide gras de l'huile de soja avec 30 molécules d'oxyde d'éthylène ou celui de l'oléylamine technique avec 15 molécules d'oxyde d'éthylène ou celui du dodécylmercaptan avec 12 molécules d'oxyde d'éthylène.

Parmi les dispersants anioniques utilisables, on peut citer par exemple le sel de sodium de l'ester sulfurique de l'alcool dodécylique, le sel

de sodium de l'acide dodécylbenzène-sulfonique, le sel de potassium ou de triéthanolamine de l'acide oléique ou de l'acide abiétique ou des mélanges de ces acides, ou le sel de sodium d'un acide sulfonique de pétrole.

Comme dispersants cationiques, on peut employer des composés d'ammonium quaternaire comme par exemple le bromure de cétyl pyridinium ou le chlorure de dioxyéthylbenzyl-dodécylammonium.

On peut également utiliser comme véhicule des agents de poudrage ou d'épendage tels que du talc, du kaolin, de la bentonite, du carbonate de calcium mais également de la farine de liège, de la farine de bois et d'autres matériaux d'origine végétale.

Les compositions peuvent également se présenter sous forme de granulés.

Les compositions selon l'invention peuvent également contenir d'autres substances susceptibles d'améliorer la dispersion, l'adhérence, la résistance à la pluie ou le pouvoir de pénétration.

Comme substances additives, on peut par exemple citer des acides gras, des résines, de la caséine ou des alginates.

Selon une forme de réalisation préférée, les compositions se présentent sous forme de solutions liquides susceptibles d'être pulvérisées à l'aide d'un gaz propulseur.

Bien que la concentration en susbstance active des compositions selon l'invention puisse varier dans de larges limites, celle-ci n'est généralement pas supérieure à 10 % environ en poids par rapport au poids total de la composition.

De préférence, ces compositions se présentent sous forme de solutions ou de suspensions contenant environ de 0,1 à 5 % en poids de la substance active .

TEST D'EVALUATION INSECTICIDE

1° Termites (Reticulitermes santonensis)

Dans un récipient en verre on place 100 termites du genre Reticulitermes santonensis et l'on pulvérise alors à une distance de 50 cm l'équivalent de 10 µg de (Hexène-5 yl)-2 (nonène-8 yl)-5 pyrrolidine à partir d'une solution de cette substance dans du pentane.

Une minute après la pulvérisation de la solution, on constate que tous les termites sont immobilisés et il en est ainsi 24 heures après la pulvérisation, ce qui démontre la puissante activité instantanée de cette substance.

2° Criquets

100 Criquets sont placés dans un récipient et l'on pulvérise à une distance d'environ 50 cm l'équivalent de 100 µg de (Hexène-5 yl)-2 (nonène-8

yl)-5 pyrrolidine à partir d'une solution de cette substance dans du pentane. On constate immédiatement après la pulvérisation une contraction du tube digestif des criquets et une immobilisation instantanée.

Sachant qu'un criquet représente de 200 à 800 fois le poids d'une termite, on peut constater que cette substance a une activité insecticide particulièrement puissante.

Des essais similaires ont été réalisés sur des punaises et on a également observé d'excellents résultats sur ces insectes.

La présente invention a également pour objet un procédé de destruction des insectes et acariens, ce procédé consistant à pulvériser ou répandre sur les lieux où prolifèrent ces insectes et acariens, une quantité efficace d'une composition contenant, dans un véhicule approprié pour une telle application, au moins un composé actif de formule (I) telle que définie ci-dessus.

Plus particulièrement, l'invention a pour objet un procédé de destruction des termites dans les structures et objets en bois, consistant à appliquer par pulvérisation ou injection dans lesdites structures et objets en bois une composition contenant, dans un véhicule approprié pour une telle application, au moins un composé actif de formule (I) telle que définie ci-dessus.

REVENDICATIONS

1. Composition insecticide et/ou acaricide caractérisée par le fait qu'elle contient dans un véhicule approprié, en tant que substance active, au moins un composé correspondant à la formule générale suivante :

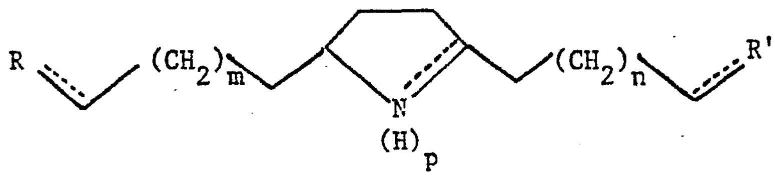

(I)

dans laquelle,

R et R', identiques ou différents, représentent un radical méthyle ou méthylène,

m et n, identiques ou différents, représentent 0 ou un nombre entier de 1 à 12,

et p est égal à 0 lorsque l'hétérocycle est la pyrroline-1 ou p est égal à 1 lorsque l'hétérocycle est la pyrrolidine,

et, les isomères et sels desdits composés de formule (I).

2. Composition selon la revendication 1, caractérisée par le fait que la substance active est au moins un composé correspondant à la formule suivante :

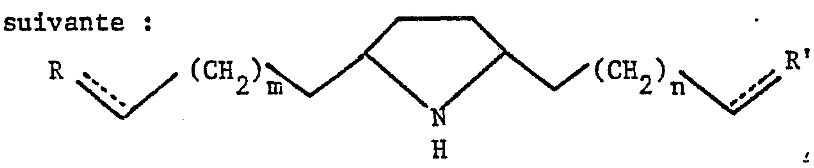

(II)

dans laquelle :

R, R', m et n ont les mêmes significations que données à la revendication 1.

3. Composition selon la revendication 2, caractérisée par le fait que la substance active est prise dans le groupe des composés suivants :

(Hexène-5 yl)-2 (nonène-8 yl)-5 pyrrolidine,

Di(nonène-8 yl)-2,5 pyrrolidine,

Di(decène-9 yl)-2,5 pyrrolidine,

(Hexène-5 yl)-2 nonyl-5 pyrrolidine,

Hexyl-2-nonyl-5 pyrrolidine,

Hexyl-2-pentyl-5 pyrrolidine,

Di hexyl-2,5 pyrrolidine, et

(Hexène-5 yl)-2 pentyl-5 pyrrolidine.

4. Composition selon la revendication 1, caractérisée par le fait que la substance active est au moins un composé correspondant à la formule suivante :

0167419

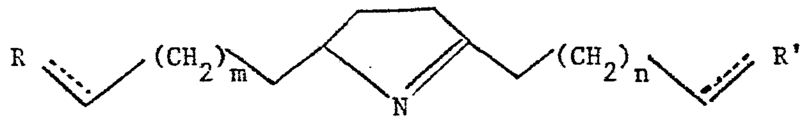

(III)

dans laquelle :

R, R', m et n ont les mêmes significations que données à la revendication 1.

5. Composition selon la revendication 4, caractérisée par le fait que la substance active est prise dans le groupe des composés suivants :

Dihydro-3,4 (hexène-5 yl)-2 (nonène-8 yl)-5, 2 H pyrrole et

Dihydro-3,4 (hexène-5 yl)-2 nonyl-5, 2 H pyrrole.

6. Composition selon l'une quelconque des revendications précédentes, caractérisée par le fait qu'elle se présente sous forme d'une émulsion, d'une suspension, d'une solution, d'une poudre ou d'un aérosol.

7. Composition selon l'une quelconque des revendications précédentes, caractérisée par le fait qu'elle contient au moins un agent dispersant pris dans le groupe constitué par les agents dispersants non-ioniques, anioniques et cationiques.

8. Composition selon l'une quelconque des revendications précédentes, caractérisée par le fait qu'elle contient la substance active à une concentration généralement pas supérieure à 10 % en poids par rapport au poids total de la composition.

9. Composition selon l'une quelconque des revendications précédentes, caractérisée par le fait qu'elle contient environ de 0,1 à 5 % de la substance active et se présente sous forme d'une solution ou d'une suspension.

10. Procédé de destruction des insectes et/ou acariens, caractérisé par le fait qu'il consiste à pulvériser ou à répandre sur les lieux ou prolifèrent ces insectes et/ou acariens, une quantité efficace d'une composition selon l'une quelconque des revendications 1 à 9.

11. Procédé selon la revendication 10 pour la destruction des termites dans les structures et objets en bois, caractérisé par le fait qu'il consiste à appliquer par pulvérisation ou injection dans lesdites structures et objets en bois au moins une composition selon l'une quelconque des revendications 1 à 9.

Office européen des brevets

0167419

Numero de la demande

# RAPPORT DE RECHERCHE EUROPEENNE

EP 85 40 0866

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int Cl 4) |
|---|---|---|---|
| X | FR-A-2 275 998 (NEDERLANDSE ORGANISATIE VOOR TOEGEPAST-NATUURWETENSCHAPPELIJK ONDERZOEK TEN BEHOEVE VAN NIJVERHEID, HANDEL EN VERKEER) * Revendications 1-4; page 3, lignes 19-26 * --- | 1-3,6-9 | A 01 N 43/36 |
| A | TETRAHEDRON, vol. 38, no. 13, 1982, pages 1949-1958, Pergamon Press Ltd., Oxford, GB; T.H. JONES et al.: "Ant venom alkaloids from Solenopsis and Monomorium species" ----- | 1-5 | |
| | | | DOMAINES TECHNIQUES RECHERCHES (Int. Cl.4) |
| | | | A 01 N |

Le présent rapport de recherche a été établi pour toutes les revendications

| Lieu de la recherche | Date d'achèvement de la recherche | Examinateur |
|---|---|---|
| LA HAYE | 16-08-1985 | DECORTE D. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503.03.82